# EUROPEAN PATENT APPLICATION

(11) **EP 3 760 266 A1**
(43) Date of publication of application: **06.01.2021**
(21) Application number: 19760200.6
(22) Date of filing: 25.02.2019
(51) Int. Cl.: A61M 21/02, A61B 5/11, A61B 5/16, A61H 1/02, A61H 7/00

(54) **SLEEPING APPARATUS AND SLEEPING SYSTEM**

(30) Priority: 28.02.2018 JP 2018035783
(71) Applicant: MTG Co., Ltd., Aichi 453-0041 (JP)
(72) Inventor: INADA Nichimu, Osaka-shi Osaka 532-0004 (JP); ISHIDO Yuta, Saihaku-gun, Tottori 689-3224 (JP); MATSUSHITA Tsuyoshi, Nagoya-shi Aichi 453-0041 (JP)
(74) Representative: Isern Patentes y Marcas S.L.
(86) International application number: PCT/JP2019/007149
(87) International publication number: WO 2019/167902

(57) **Abstract**

The present invention provides a sleeping apparatus which can improve the quality of sleep, by evaluating the quality of human sleep and presenting the control methods of the exercise forcing means based on the evaluation result. The sleeping apparatus comprises a body support part which supports the user's body, a sleep information detection means which detects said user's sleep information, and a memory part which stores said user's sleep information, wherein the sleeping apparatus comprises an exercise forcing means which forces said user's body to exercise, a sleep state evaluation means which evaluates said user's sleep state based on said user's sleep information, and a control means which can output control methods of said exercise forcing means based on the evaluation result from said sleep state evaluation means.

## Description

### BACKGROUND OF THE INVENTION

### [Field of the Invention]

The present invention relates to a sleeping apparatus and a sleeping system, in particular, a sleeping apparatus that can improve the quality of sleep, by evaluating the quality of human sleep and presenting control methods of an exercise forcing means based on the evaluation result.

### Description of Related Art

Sleep is said to be a barometer of health, and sleeping comfortably and waking up in a good mood let people feel brisk and fit when they wake up, which is often experienced in daily life. On the other hand, in case people suffer from or carry a propensity for insomnia, or are forced to sleep in the daytime due to late-night work etc., they might often find themselves feeling awful when they wake up. In fact, irrespective of consciously or subconsciously, the sleep state will affect people's mood and actions when they wake up, and subsequently rules the quality of their activities in the daytime when they are awake.

Here, a sleep evaluation device comprising a sleep information evaluation means, an advice means, and a presentation means is disclosed (for example, refer to Japanese Unexamined Patent Application Publication No. 2001-61819). The sleep information evaluation means evaluates a sleeper's sleep state based on the biological information measured during sleep. The advice means generates advice about actions for the sleeper to take by the sleeper's next sleep while the sleeper is awake, based on the evaluation result from the sleep information evaluation means. The presentation means presents the evaluation result and advice. The sleep information evaluation means provides the sleeper with advice about exercise etc., for example, recommending exercise or stretching before sleeping, as information for improving the sleeper's sleep state.

### BRIEF SUMMARY OF THE INVENTION

However, although the advice as described above is given by the sleep evaluation device, the user, provided with advice of recommending exercise or stretches before sleeping, sometimes did not understand what kind of exercise or stretches to do, failed to carry out appropriate exercise or stretches, and ended up finding it difficult to improve the quality of sleep.

The present invention is made in view of problems described above, and aims to provide a sleeping apparatus and a sleeping system that can improve the quality of sleep, by evaluating the quality of humans' sleep and presenting control methods of exercise forcing means based on the evaluation result.

In order to achieve the above purposes, the sleeping apparatus in the present invention comprises, a body support part which supports a user's body, a sleep information detection means which detects said user's sleep information and a memory part which stores said user's sleep information, wherein said sleeping apparatus comprises an exercise forcing means which forces said user's body to exercise, a sleep state evaluation means which evaluates said user's sleep state based on said user's sleep information, and a control means which can output control methods of said exercise forcing means based on the evaluation result from said sleep state evaluation means.

According to the sleeping apparatus in the present invention, the sleep information detection means detects the user's sleep information and stores it in the memory part. The sleep state evaluation means evaluates the user's sleep state based on the user's sleep information. And the control means outputs control methods of the exercise forcing means based on the evaluation result. As control methods of the exercise forcing means for example, the control means recommends an appropriate stretch course, displays it on a display device (output of image signals), provides it by audio guide (output of audio signals) and performs it automatically (output of control signals). In this configuration, the user can have the user's body exercise, by controlling the exercise forcing means based on an appropriate stretch course etc. as output control methods of the exercise forcing means. As a result, comfortable sleep is provided by the user doing stretches before falling into a sleep, and a vigorous wake-up is provided by the user doing stretches after waking up.

In the above invention, it is preferable that said body support part is a mattress having an upper surface on which said user lies.

In the above invention, it is preferable that said sleep information detection means is a microphone which detects noise generated from said user and/or a body motion sensor which detects body motions of said user.

In the above invention, it is preferable that the control methods of said exercise forcing means are displayed on a display device and/or provided by audio guide.

In the above invention, it is preferable that said exercise forcing means has multiple air cells and a pump connected to the multiple air cells.

In the above invention, it is preferable that said memory part stores multiple stretch courses with different contents of stretches and that said control means selects one stretch course from the multiple stretch courses and recommends the selected stretch course.

In the above invention, it is preferable that said memory part stores multiple stretch courses with different contents of stretches and that said control means selects one stretch course from the multiple stretch courses and controls said exercise forcing means based on the selected stretch course.

In the above invention, it is preferable that said memory part stores a scheduled wake-up time of said user and that said control means operates said exercise forcing means to perform the motions to wake up said user when said user is in the optimal state to wake up based on said evaluation result, before said scheduled wake-up time.

In the above invention, it is preferable that said control means controls said exercise forcing means before the user falls into a sleep and/or after the user wakes up.

In the above invention, it is preferable that said control means has AI function and controls said exercise forcing means based on said user's sleep information accumulated in said memory part.

Also, in order to achieve the above purposes, the sleeping system in the present invention comprises, a sleeping apparatus with a body support part which supports the user's body and a sleep information detection means which detects said user's sleep information, and a memory part which stores said user's sleep information, wherein said sleeping apparatus comprises an exercise forcing means which forces said user's body to exercise, and the sleeping system comprises a sleep state evaluation means which evaluates said user's sleep state based on said user's sleep information and a control means which can output control methods of said exercise forcing means based on the evaluation result from said sleep state evaluation means.

According to the sleeping system in the present invention, the sleep information detection means detects the user's sleep information and stores it in the memory part. The sleep state evaluation means evaluates the user's sleep state based on the user's sleep information. And the control means outputs control methods of the exercise forcing means based on the evaluation result. As control methods of the exercise forcing means for example, the control means recommends an appropriate stretch course, displays it on a display device (output of image signals), provides it by audio guide (output of audio signals) and performs it automatically (output of control signals). In this configuration, the user can have the user's body exercise, by controlling the exercise forcing means based on an appropriate stretch course etc. as output control methods of the exercise forcing means. As a result, comfortable sleep is provided by the user doing stretches before falling into a sleep, and a vigorous wake-up is provided by the user doing stretches after waking up.

In the above invention, it is preferable that said body support part is a mattress having an upper surface on which said user lies.

In the above invention, it is preferable that said sleep information detection means is a microphone which detects noise generated from said user and/or a body motion sensor which detects body motions of said user.

In the above invention, it is preferable that said exercise forcing means has multiple air cells and a pump connected to the multiple air cells.

In the above invention, it is preferable that the control methods of said exercise forcing means are displayed on a display device and/or provided by audio guide.

In the above invention, it is preferable that said memory part stores multiple stretch courses with different contents of stretches and that said control means selects one stretch course from the multiple stretch courses and recommends the selected stretch course.

In the above invention, it is preferable that said memory part stores multiple stretch courses with different contents of stretches and that said control means selects one stretch course from the multiple stretch courses and controls said exercise forcing means based on the selected stretch course.

In the above invention, it is preferable that said memory part stores a scheduled wake-up time of said user and that said control means operates said exercise forcing means to perform the motions to wake up said user when said user is in the optimal state to wake up based on said evaluation result, before said scheduled wake-up time.

In the above invention, it is preferable that said control means controls said exercise forcing means before the user falls into a sleep and/or after the user wakes up.

In the above invention, it is preferable that said control means has AI function and controls said exercise forcing means based on said user's sleep information accumulated in said memory part.

### [Effect of the Invention]

According to a sleeping apparatus and a sleeping system of the present invention, it is possible to improve the quality of sleep, by evaluating the quality of human sleep (suitable sleep for a user) and presenting control methods of exercise forcing means based on the evaluation result. Particularly, comfortable sleep is provided by the user doing stretches before falling into a sleep, and a vigorous wake-up is provided by the user doing stretches after waking up.

### BRIEF DESCRIPTION OF THE SEVERAL VIEWS OF THE DRAWING(S)

[FIG. 1] FIG. 1 is a perspective view showing the main composition of a sleeping apparatus in the embodiment 1 of the present invention.
[FIG. 2] FIG. 2 is a block diagram showing the composition of the sleeping apparatus in the embodiment 1 of the present invention.
[FIG. 3] FIG. 3 is an exploded perspective view of a mattress.
[FIG. 4] FIG. 4 is a figure for describing the arrangement of the air cells in the mattress.
[FIG. 5] FIG. 5 is a sectional view of the mattress which is folded in three.
[FIG. 6] FIG. 6 is a figure for describing stretch courses.
[FIG. 7] FIG. 7 is a figure for describing a "Good morning course".
[FIG. 8] FIG. 8 is a figure for describing a "Good morning course".
[FIG. 9] FIG. 9 is a flow chart for describing the usage of the sleeping apparatus.
[FIG. 10] FIG. 10 is a perspective view showing the main composition of the sleeping system in the embodiment 2 of the present invention.

### DETAILED DESCRIPTION OF THE INVENTION

The embodiments of the present invention are described below with reference to drawings.

### (Embodiment 1)

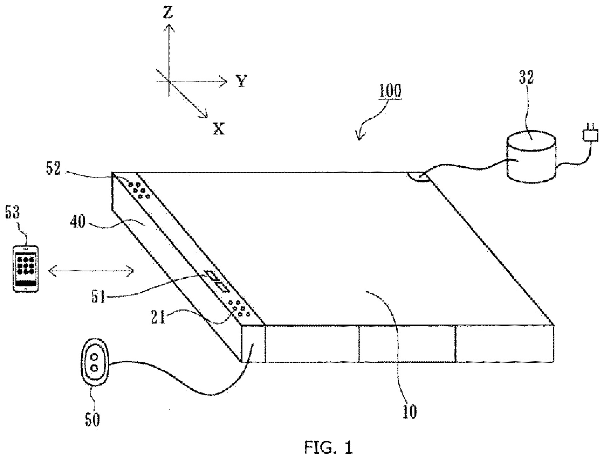

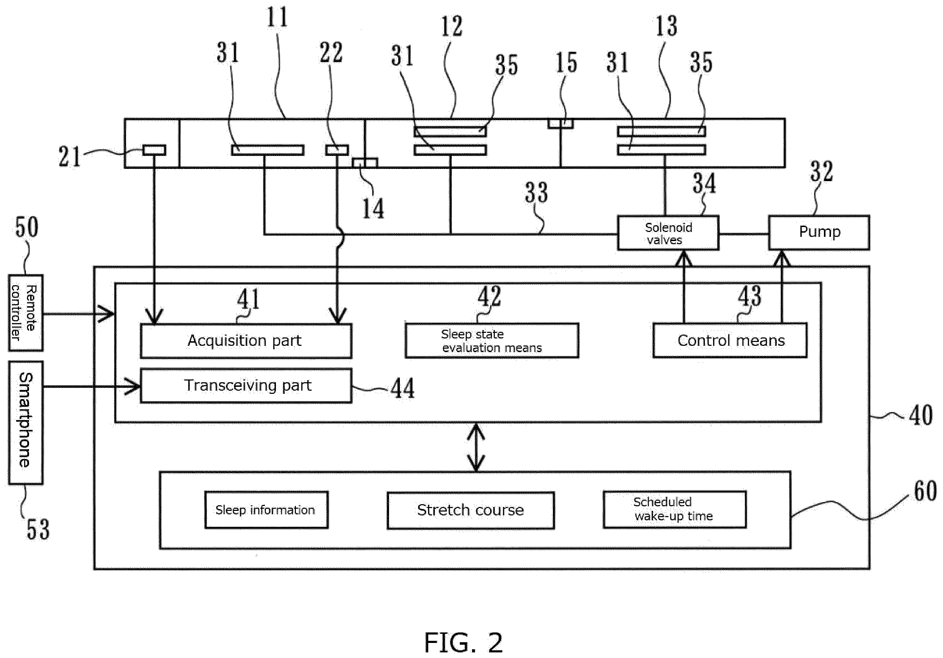

FIG. 1 is a perspective view showing the main composition of a sleeping apparatus in the embodiment 1 of the present invention, and FIG. 2 is a block diagram showing the composition of the sleeping apparatus in the embodiment 1 of the present invention. Here, the vertical direction (up-down direction) is Z-direction, one direction perpendicular to Z-direction (left-right direction) is X-direction, and the other direction perpendicular to Z-direction and X-direction (front-back direction) is Y-direction.

The sleeping apparatus 100 comprises a mattress (a body support part) 10, a sleep information detection means including a microphone 21 and a body motion sensor 22, an exercise forcing means including multiple (any number of) air cells 31 and a pump (a cylindrical shaped pump box) 32, and a control unit 40 to control the entire sleeping apparatus 100.

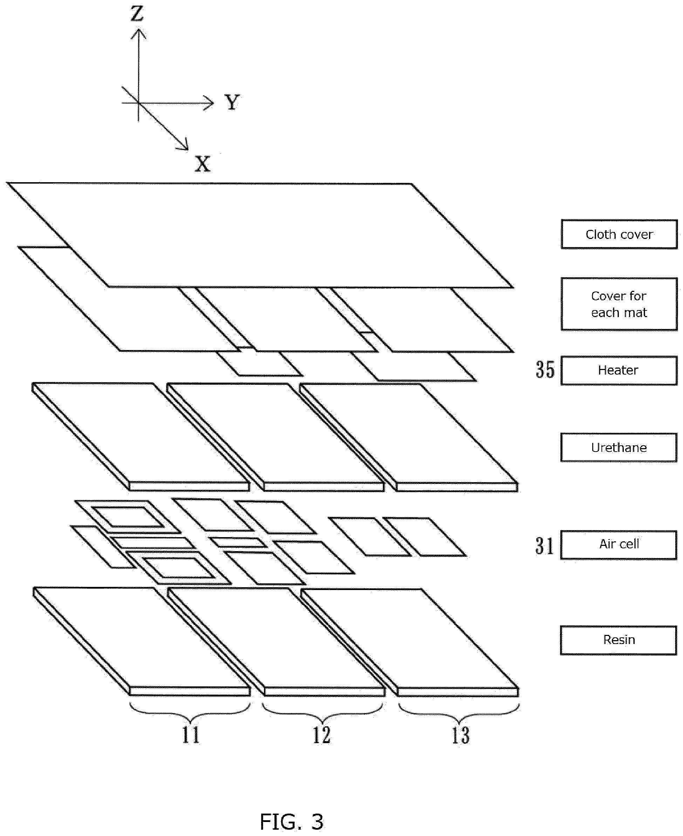

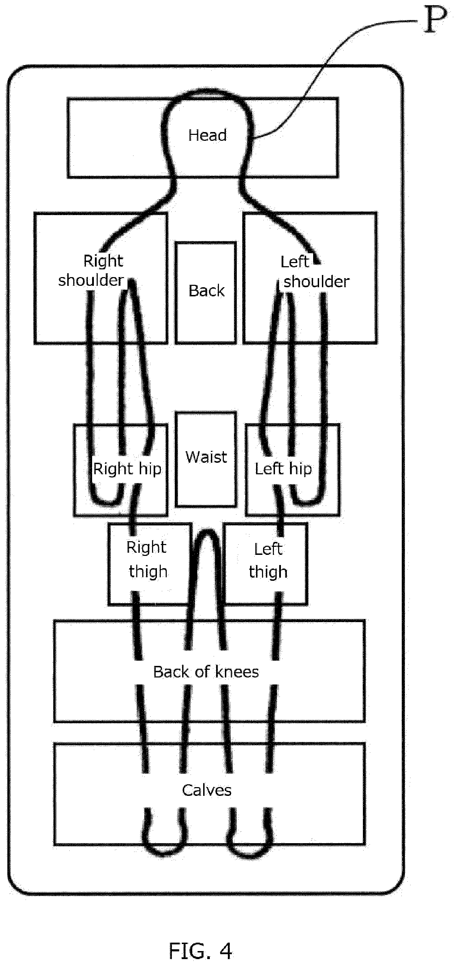

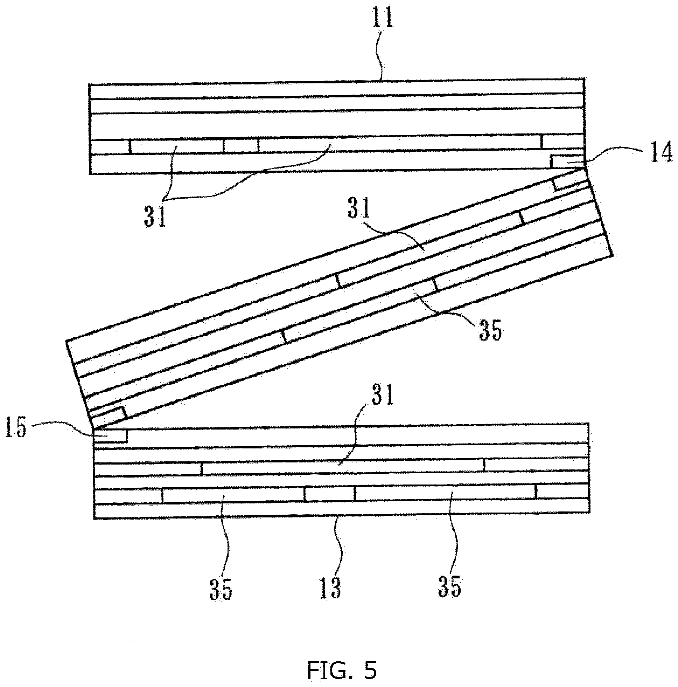

First, a mattress 10 having an upper surface on which a user P lies, is described. FIG. 3 is an exploded perspective view of the mattress, FIG. 4 is a figure for describing the arrangement of the air cells in the mattress, and FIG. 5 is a sectional view of the mattress folded in three.

The mattress 10 is divided into three, namely a head-shoulder mat part 11, a body mat part 12, and a leg mat part 13, and possible to fold in three as there are two connections (notch structures) formed on the lower surface between the head-shoulder mat part 11 and the body mat part 12 and another two connections (notch structures) on the upper surface between the body mat part 12 and the leg mat part 13.

The leg mat part 13 is laminated by a resin layer, a urethane layer, a cover layer and a cloth cover layer, with one heater 35 placed between the urethane layer and the cover layer and two air cells 31 placed between the resin layer and the urethan layer, and each air cell is connected to an externally placed pump 32 via air tubes 33 and solenoid valves 34.

And as shown in FIG. 4, when a mattress 10 is unfolded and a user P lies on his back on the upper surface of the mattress, one air cell 31 comes under the back of both knees of the user P, and one air cell 31 comes under both calves of the user P.

Piping, such as air tubes 33 and electric cables (not shown), is placed in a pocket or the like provided on the peripheral edge on the upper surface of the resin layer, and connected so that it would not get damaged when the mattress is folded by the notches 14, 15.

The body mat part 12, too, is laminated by a resin layer, a urethane layer, a cover layer and a cloth cover layer, but with one heater 35 placed between the urethane layer and the cover layer and five air cells 31 placed between the resin layer and the urethan layer, and each air cell is connected to the externally placed pump 32 via air tubes 33 and solenoid valves 34.

And as shown in FIG. 4, when a mattress 10 is unfolded and a user P lies on his back on the upper surface of the mattress, one air cell 31 comes under the user's (P's) waist, one air cell 31 comes under the user's (P's) right hip, one air cell 31 comes under the user's (P's) left hip, one air cell 31 comes under the user's (P's) right thigh, and one air cell 31 comes under the user's (P's) left thigh.

Piping, such as air tubes 33 and electric cables (not shown), is placed in a pocket or the like provided on the peripheral edge on the upper surface of the resin layer, and connected so that it would not get damaged when the mattress is folded by the notches 14, 15.

The head-shoulder mat part 11, too, is laminated by a resin layer, a urethane layer, a cover layer and a cloth cover layer, but with one body motion sensor 22 placed between the resin layer and the urethan layer and six air cells 31 placed between the resin layer and the urethan layer, and each air cell is connected to the externally placed pump 32 via air tubes 33 and solenoid valves 34.

And as shown in FIG. 4, when a mattress 10 is unfolded and a user P lies on his back on the upper surface of the mattress, one air cell 31 comes under the user's (P's) head, one air cell 31 comes under the user's (P's) back, two air cells 31 come stacked together under the user's (P's) right shoulder, and two air cells 31 come stacked together under the user's (P's) left shoulder.

Piping, such as air tubes 33 and electric cables (not shown), is placed in a pocket or the like provided on the peripheral edge on the upper surface of the resin layer, and connected so that it would not get damaged when the mattress is folded by the notches 14, 15.

Also, one body motion sensor 22 is a pressure sensor of airbag type which reads out changes in air pressure, is strip-shaped extending from left edge to right edge in X-direction and is placed under the user P between the user's back and waist. In this configuration, the user's (P's) body motions during sleep (subtle changes due to body motions, vibrations, respiration rate, heart rate, or pulse rate) are detected, and the body motion information (sleep information) is output to a control unit 40 described later.

Furthermore, a microphone 21 is placed at the front end of the head-shoulder mat part 11. In this configuration, noise generated by the user P during sleep (sleep information, such as snoring) is detected and the noise generated by the user P (sleep information) is output to the control unit 40 described later. The microphone 21 may be installed in a separate device, such as a smartphone, and, in this case, is favorably placed high above the user P, which makes noise collection easier. Also, the microphone 21 may be a microphone for a speech recognition function. In this configuration, the microphone can also be operated instead of the remote controller 50 described later.

Besides, the control unit 40 is built in the front end of the head-shoulder mat part 11, and is connected with a remote controller 50 where the user P can perform input operation using START button and STOP button or the like. Also, there are two USB ports 51 formed on the control unit 40 through which the control unit can be connected with external devices. Furthermore, there is a speaker 52 with speaking function formed on the control unit 40. In this configuration, it is possible to provide the stretch courses etc. described later by audio guide with the speaker 52.

The control unit 40 comprises CPU and memory (storage part) 60 etc., and controls the entire sleeping apparatus 100.

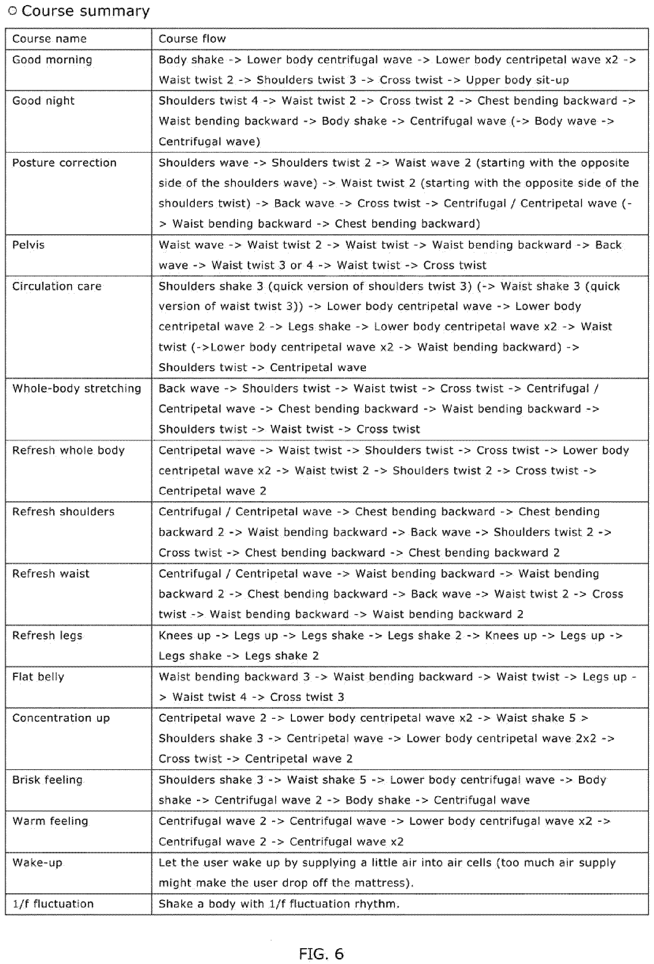

The memory 60 in the embodiment 1 is configured to store the stretch courses-related data, and stores multiple (any number of) stretch courses downloaded by a smartphone or the like. These stretch courses are the programs created by the sleeping apparatus 100 manufacturer etc., which operate each air cell 31 and each heater 35 in the predetermined procedures. As stretch courses, for example, "Good morning course", "Good night course", "Posture correction course" and "Pelvis course" etc. are prepared, as shown in FIG. 6.

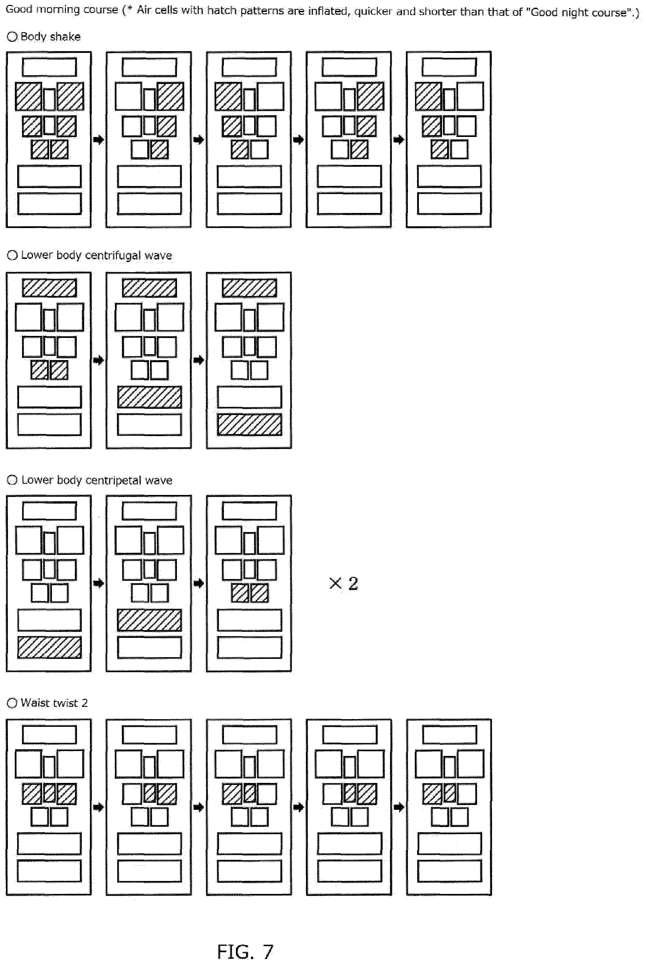

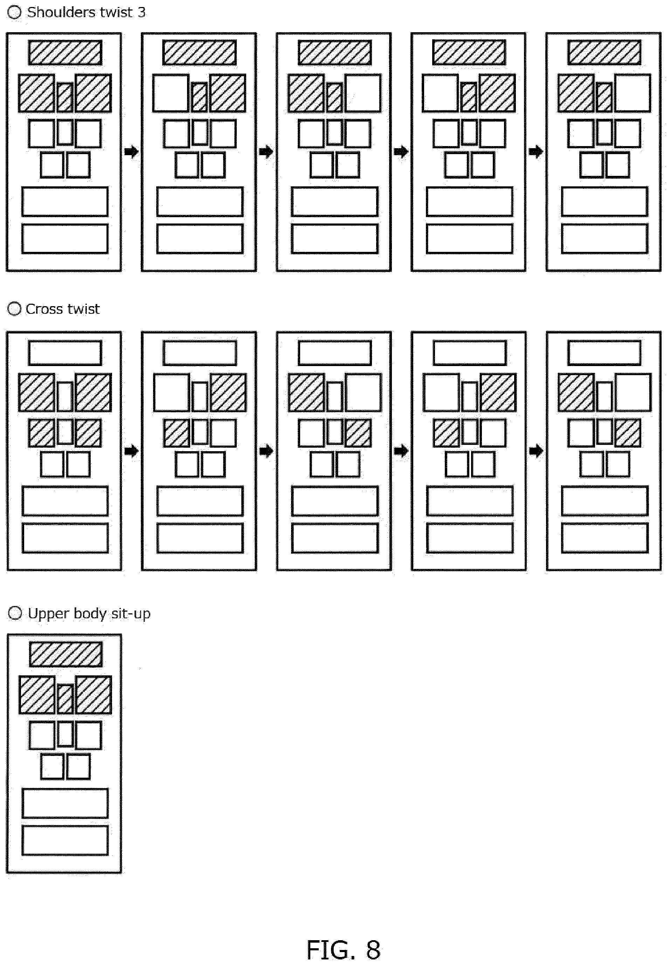

Now, "Good morning course", one of the stretch courses, is described. FIG. 7 and FIG. 8 are figures for describing the "Good morning course". It should be noted that those air cells with hatch patterns represent air cells being inflated and those air cells without hatch patterns represent the air cells not being inflated.

In "Good morning course", as shown in FIG. 6, "Body shake", "Lower body centrifugal wave", "Lower body centripetal wave" x2, "Waist twist 2", "Shoulders twist 3", "Cross twist", and "Upper body sit-up" are performed in sequence.

In "Body shake" as shown in FIG. 7, air cells 31 under right shoulder, left shoulder, right hip, left hip, right thigh, and left thigh are inflated in time t1, air cells 31 under left shoulder, left hip, and left thigh are inflated in t2, air cells 31 under right shoulder, right hip, and right thigh are inflated in t3, air cells 31 under left shoulder, left hip, and left thigh are inflated in t4, and air cells 31 under right shoulder, right hip, and right thigh are inflated in t5. In this configuration, a stretch to shake a body is performed for the user P.

It should be noted that descriptions about "Lower body centrifugal wave", "Lower body centripetal wave" x2, "Waist twist 2", "Shoulders twist 3", "Cross twist", and "Upper body sit-up" are omitted.

With such a sleeping apparatus (100), the user P lies on the upper surface of the mattress 10, sets a scheduled wake-up time using a remote controller 50 before sleeping, and selects "Good night course", and then "Good night course" is performed. Then, when the user P has fallen into a sleep on the mattress, the control unit 40 collects the user's (P's) sleep information from the microphone 21 and the body motion sensor 22, evaluates the user's (P's) sleep state, and automatically selects one stretch course from multiple stretch courses in order to perform the selected stretch course when the user P wakes up and presses the START button of the remote controller 50.

The control unit 40 comprises, specifically, an acquisition part 41 which acquires the user's (P's) sleep information from the microphone 21 and the body motion sensor 22 and stores it in the memory 60, a sleep state evaluation means 42 which evaluates the user's (P's) sleep state, a control means 43 which can output the control methods of the exercise forcing means, and a transceiving part 44 which can send and receive data to/from smartphones 53 or the like.

The acquisition part 41 acquires the noise generated from the user P (sleep information) by the microphone 21 at a predetermined time interval and stores it in the memory 60 as well as acquires the user's (P's) body motion information (sleep information) by the body motion sensor 22 at a predetermined time interval and stores it in the memory 60. In this configuration, the memory stores the user's (P's) sleep information during sleep throughout the day, and at the same time, accumulates the user's (P's) sleep information during sleep in the past.

The sleep state evaluation means 42 evaluates the user's (P's) sleep state based on the noise (sleep information) generated from the user P and the user's (P's) body motion information (sleep information) stored in the memory 60. For example, with the noise (sleep information) generated from the user P and the user's (P's) body motion information (sleep information), bedtime, sleep-onset time, sleep latency, awakening time, wake-up time, getting-up latency, halfway-awakening time, halfway-awakening frequency, total sleep hours, rollover frequency, body motion frequency, apneic period, respiration rate, pulse rate, and heart rate are calculated as the user's sleep state, by for example a Kernel method. In this instance, it is possible to evaluate the sleep state only by the generated noise (sleep information), to evaluate the sleep state only by the user's (P's) body motion information (sleep information), or to accurately evaluate the sleep state by the generated noise (sleep information) as well as the user's (P's) body motion information (sleep information).

Bedtime is the time when the user P goes to bed. Sleep-onset time is the time when the user P falls into a sleep. Sleep latency is the time duration from when the user P goes to bed until the user falls into a sleep. Awakening time is the time when the user P eventually gets awake. Wake-up time is the time when the user P eventually gets out of bed. Getting-up latency is the time duration from when the user P gets awake until the user (P) eventually gets out of bed. Halfway-awakening time is the sum of the awakening time between the sleep-onset time and the awakening time.
Halfway-awakening frequency is the sum of the awakening frequency between the sleep-onset time and the awakening time. Total sleep hours are the time subtracting the halfway-awakening time from the time duration between the sleep-onset time and the awakening time. Rollover frequency is the frequency of rollovers during the time duration between the sleep-onset time and the awakening time excluding the halfway-awakening time. Body motion frequency is the frequency of the body motions (major body motions and minor body motions) during the time duration between the sleep-onset time and the awakening time excluding the halfway-awakening time. Apneic period is the sum of the apneic time between the sleep-onset time and the awakening time. Respiration rate is a rate of respiration of the user P during sleep. Pulse rate is a rate of pulse of the user P during sleep. Heart rate is a rate of heartbeat of the user P during sleep.

A control means 43 selects one stretch course from multiple stretch courses based on the evaluation result from the sleep state evaluation means 42, and based on the selected stretch course, controls the exercise forcing means including multiple air cells 31 and a pump 32.

For example, when the sleep state evaluation means 42 determines a sleep to be a deep sleep based on the accumulated sleep information of the user P, "Good morning course" will be selected as an appropriate stretch course, so that the selected "Good morning course" will be performed when the user P wakes up and presses the START button on the remote controller 50. On the other hand, when the sleep state evaluation means 42 determines a sleep to be a shallow sleep based on the accumulated sleep information of the user P, "Brisk feeling course" will be selected as an appropriate stretch course, so that the selected "Brisk feeling course" will be performed when the user P wakes up and presses the START button on the remote controller 50.

The control means 43 may have AI function, based on which one stretch course may be selected from the multiple stretch courses.

Also, a control means 43 performs "Good morning wave" (wake-up motions) slightly before (before) the scheduled wake-up time set in the memory 60 (when the user P is in the optimal state to wake up based on the evaluation result), and stops "Good morning wave" when the user P wakes up and presses the STOP button on the remote controller 50. In this instance, when the START button on the remote controller 50 is subsequently pressed, the selected stretch course will be performed. In other words, the control means has the exercise forcing means perform the wake-up motions and then the stretch motions.

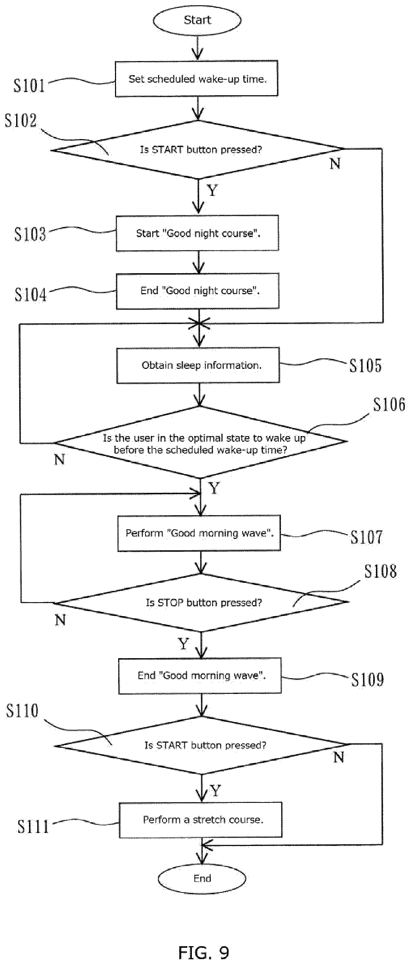

Next, an example of the usages of the sleeping apparatus 100 in the embodiment 1 is described. FIG. 9 is a flow chart for describing the usage of the sleeping apparatus.

Firstly, lying on the upper surface of the mattress 10, the user P sets a scheduled wake-up time (7 o'clock for weekdays, for example) using the remote controller 50 before sleeping (Step S101). It is also possible that a scheduled wakeup time for everyday (7 o'clock, for example) is preset in the sleeping apparatus 100.

Secondly, it is determined whether or not the START button on the remote controller 50 has been pressed, in order for the user P to perform a stretch for comfortable sleep before falling into a sleep (Step S102). When it is determined that the START button has been pressed, "Good night course" selected with the remote controller 50 will be performed (Step S103).

Once "Good night course" has been performed for the predetermined time (7-10 minutes), "Good night course" will be ended (Step S104). This stimulates the user's (P's) parasympathetic nerve system and induces sleep onset.

When the process of Step S104 is completed or when it is determined that the START button has not been pressed in the process of Step S102 (which means the user has fallen into a sleep without doing any stretches), once the user P has fallen into a sleep, the noise generated from the user P (sleep information) will be obtained by a microphone 21 at a predetermined interval and stored in the memory 60, and at the same time, the body motion information of the user P (sleep information) will be obtained by a body motion sensor 22 at a predetermined interval and stored in the memory 60 (Step S105).

Next, before the set scheduled wake-up time (at 6:50 for example), the control means 43 determines whether or not the optimal state for the user P to wake up has been achieved, based on the sleep information (Step S106). When it is determined that the optimal state has not been achieved yet, the process of Step S105 will be repeated.

When it is determined that the optimal state has been achieved, on the other hand, the control means 43 will perform "Good morning wave" to have the body move from head to legs in sequence (wake-up motion), rather than the stretch motions twisting the body, in order to wake up the user P from sleep (Step S107).

The control means 43 determines whether or not the STOP button on the remote controller 50 has been pressed when the user P wakes up (Step S108). When it is determined that the STOP button has not been pressed yet, the process of Step S107 will be repeated.

When it is determined that the STOP button has been pressed, on the other hand, "Good morning wave" will be stopped (Step S109).

Next, the control means 43 determines whether or not the START button on the remote controller 50 has been pressed (Step S110). When it is determined that the START button has been pressed, the sleep state evaluation means 42 will evaluate the user's (P's) sleep state based on the noise generated from the user P (sleep information) and the user's (P's) body motion information (sleep information) stored in the memory 60, and the control means 43 will select one stretch course from the multiple stretch courses and perform the selected stretch course, so as to provide the user P with a vigorous wake-up (Step Sill). This stimulates the user's (P's) parasympathetic nerve system and offers a brisk wake-up.

When the process of Step S111 is completed or when it is determined that the START button has not been pressed in the process of Step S110 (which means the user has not done any stretches due to no time available in the morning), this flow chart will be ended.

According to the sleeping apparatus 100 in the embodiment 1 as above, it is possible to improve the quality of sleep, by evaluating the quality of sleep of the user P (a sleep suitable for the user P) and presenting the control methods of the exercises forcing means (the optimal stretch course) based on the evaluation result. Particularly, comfortable sleep is provided by the user P doing stretches before falling into a sleep, and a vigorous wake-up is provided by the user P doing stretches after waking up.

### (Embodiment 2)

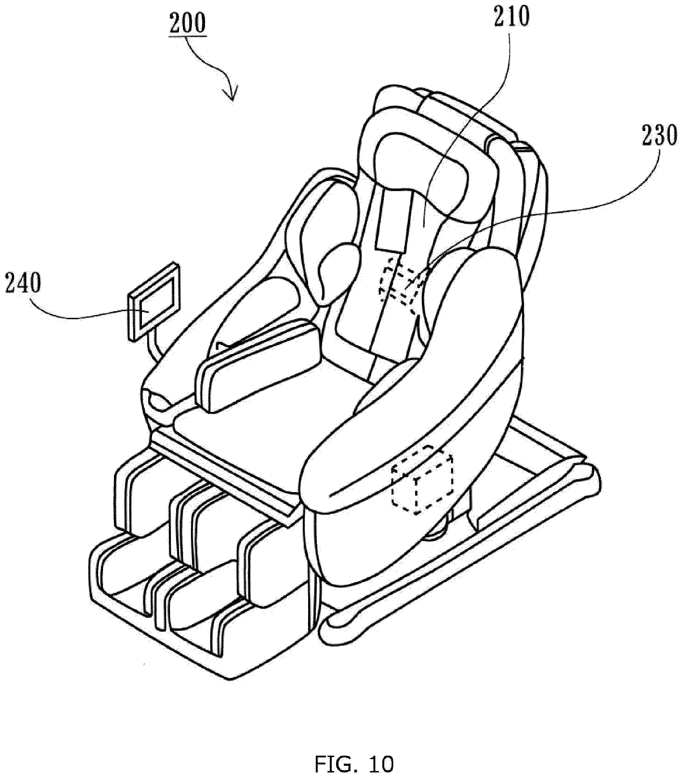

FIG. 10 is a perspective view showing the main composition of the sleeping system in the embodiment 2 of the present invention. The same numbers are given to the same components as the ones in the sleeping apparatus 100 described above.

A sleeping system 200 comprises a reclining chair (a body support part) 210, a sleep information detection means (not shown), an exercise forcing means 230, and a control unit 240 which controls the entire sleeping system 200.

A control unit 240 has a display screen on its surface, and in its inside, an acquisition part which acquires the user's (P's) sleep information and stores it in the memory, a sleep state evaluation means which evaluates the user's (P's) sleep state, and a control means which outputs the control methods of the exercise forcing means.

In other words, the control unit 240 is a separate device from the reclining chair (body support part) 210.

The control means of the control unit 240 selects one stretch course from the multiple stretch courses based on the evaluation result from the sleep state evaluation means, and recommends the selected stretch course.

For example, when the sleep state evaluation means determines a sleep to be a deep sleep based on the accumulated sleep information of the user P, the recommendation of "Good morning course" will be displayed on the display screen as an appropriate stretch course. On the other hand, when the sleep state evaluation means determines a sleep to be a shallow sleep based on the accumulated sleep information of the user P, "Brisk feeling course" will be selected as an appropriate stretch course and the recommendation of the selected "Brisk feeling course" will be displayed on the display screen.

In this configuration, the user P selects a stretch course to perform while observing the display screen.

According to the sleeping system 200 in the embodiment 2 as above, it is possible to improve the quality of sleep, by evaluating the quality of sleep of the user P (a sleep suitable for the user P) and presenting the control methods of the exercises forcing means (the optimal stretch course) based on the evaluation result. Particularly, comfortable sleep is provided by the user P doing stretches before falling into a sleep, and a vigorous wake-up is provided by the user P doing stretches after waking up.

### (Other embodiments)

(1) In the sleeping apparatus 100 in the embodiment 1, the body support part has been described to be a mattress 10, but is not limited to it. The body support part may be, for example, a reclining chair or the like.
(2) In the sleeping apparatus 100 in the embodiment 1, the exercise forcing means has been described to comprise multiple air cells 31 and a pump 32, but is not limited to them. The exercise forcing means may comprise, for example, treatment elements and an actuator or the like.
(3) In the sleeping apparatus 100 in the embodiment 1, the sleep information detection means has been described to comprise a microphone 21 and a body motion sensor 22, but is not limited to them. The sleep information detection means may comprise, for example, a thermometer, an electroencephalograph or an ultrasonic detector or the like.
(4) In the sleeping apparatus 100 in the embodiment 1, it has been described that one stretch course is selected from the multiple stretch courses and that the selected stretch course is performed in the process of Step S111, when it is determined that the START button has been pressed, but is not limited to them. It may be configured so that one stretch course is selected from the multiple stretch courses and that the selected stretch course is performed in the process of Step S102, when it is determined that the START button has been pressed.

### [Industrial availability]

The sleeping apparatus in the present invention is useful as a sleeping apparatus which can improve the quality of sleep, by evaluating the quality of human sleep and presenting the control methods of the exercise forcing means based on the evaluation result.

### [Description of symbols]

- 10: Mattress (Body support part)
- 21: Microphone (Sleep information detection means)
- 22: Body motion sensor (Sleep information detection means)
- 31: Air cell (Exercise forcing means)
- 32: Pump (Exercise forcing means)
- 40: Control unit
- 41: Acquisition part
- 42: Sleep state evaluation means
- 43: Control means
- 44: Transceiving part
- 100: Sleeping apparatus

## Claims

1. A sleeping apparatus, comprising:
a body support part which supports a user's body;
a sleep information detection means which detects said user's sleep information; and
a memory part which stores said user's sleep information,
wherein said sleeping apparatus comprises:
an exercise forcing means which forces said user's body to exercise;
a sleep state evaluation means which evaluates said user's sleep state based on said user's sleep information; and
a control means which can output control methods of said exercise forcing means based on the evaluation result from said sleep state evaluation means.

2. The sleeping apparatus according to claim 1, wherein said body support part is a mattress having an upper surface on which said user lies.

3. The sleeping apparatus according to claim 1 or 2, wherein said sleep information detection means is a microphone which detects noise generated from said user and/or a body motion sensor which detects body motions of said user.

4. The sleeping apparatus according to any one of the claims 1-3, wherein said exercise forcing means has multiple air cells and a pump connected to the multiple air cells.

5. The sleeping apparatus according to any one of the claims 1-4, wherein said control means displays the control methods of said exercise forcing means on a display device and/or provides the control methods of said exercise forcing means by audio guide.

6. The sleeping apparatus according to any one of the claims 1-4, wherein said memory part stores multiple stretch courses with different contents of stretches, and
said control means selects one stretch course from the multiple stretch courses and recommends the selected stretch course.

7. The sleeping apparatus according to any one of the claims 1-4, wherein said memory part stores multiple stretch courses with different contents of stretches, and
said control means selects one stretch course from the multiple stretch courses and controls said exercise forcing means based on the selected stretch course.

8. The sleeping apparatus according to any one of the claims 1-7, wherein said memory part stores a scheduled wake-up time of said user, and
said control means operates said exercise forcing means to perform the motions to wake up said user when said user is in the optimal state to wake up based on said evaluation result, before said scheduled wake-up time.

9. The sleeping apparatus according to any one of the claims 1-7, wherein said control means controls said exercise forcing means before said user falls into a sleep and/or after said user wakes up.

10. The sleeping apparatus according to any one of the claims 1-9, wherein the control means has AI function, and
controls said exercise forcing means based on said user's sleep information accumulated in said memory part.

11. A sleeping system, comprising:
a sleeping apparatus with a body support part which supports the user's body and a sleep information detection means which detects said user's sleep information; and
a memory part which stores said user's sleep information,
wherein said sleeping apparatus comprises an exercise forcing means which forces said user's body to exercise, and
wherein said sleeping system comprises:
a sleep state evaluation means which evaluates said user's sleep state based on said user's sleep information; and
a control means which can output control methods of said exercise forcing means based on the evaluation result from said sleep state evaluation means.

12. The sleeping system according to claim 11, wherein said body support part is a mattress having an upper surface on which said user lies.

13. The sleeping system according to claim 11 or 12, wherein said sleep information detection means is a microphone which detects noise generated from said user and/or a body motion sensor which detects body motions of said user.

14. The sleeping system according to any one of the claims 11-13, wherein said exercise forcing means has multiple air cells and a pump connected to the multiple air cells.

15. The sleeping system according to any one of the claims 11-14, wherein said control means displays the control methods of said exercise forcing means on a display device and/or provides the control methods of said exercise forcing means by audio guide.

16. The sleeping system according to any one of the claims 11-14, wherein said memory part stores multiple stretch courses with different contents of stretches, and
said control means selects one stretch course from the multiple stretch courses and recommends the selected stretch course.

17. The sleeping system according to any one of the claims 11-14, wherein said memory part stores multiple stretch courses with different contents of stretches, and
said control means selects one stretch course from the multiple stretch courses and controls said exercise forcing means based on the selected stretch course.

18. The sleeping system according to any one of the claims 11-17, wherein said memory part stores a scheduled wake-up time of said user, and
said control means operates said exercise forcing means to perform the motions to wake up said user when said user is in the optimal state to wake up based on said evaluation result, before said scheduled wake-up time.

19. The sleeping system according to any one of the claims 11-17, wherein said control means controls said exercise forcing means before said user falls into a sleep and/or after said user wakes up.

20. The sleeping system according to any one of the claims 11-19, wherein the control means has AI function, and
controls said exercise forcing means based on said user's sleep information accumulated in said memory part.
